# EUROPEAN PATENT APPLICATION

(11) **EP 1 808 141 A1**
(43) Date of publication of application: **18.07.2007**
(21) Application number: 06000496.7
(22) Date of filing: 11.01.2006
(51) Int. Cl.: A61B 17/70, A61B 17/00

(54) **Bone anchoring assembly**

(71) Applicant: BIEDERMANN MOTECH GmbH, 78054 VS-Schwenningen (DE)
(72) Inventor: Harms, Jürgen, Prof., 76227 Karlsruhe (DE); Matthis, Wilfried, 79367 Weisweil (DE); Rapp, Helmar, 78652 Deisslingen (DE); Biedermann, Lutz, 78048 VS-Villingen (DE)
(74) Representative: Hofer, Dorothea

(57) **Abstract**

A bone anchoring assembly for use in spinal surgery or trauma surgery is provided comprising
an anchoring element (1) having a shaft (2) and a receiving part (3) having a substantially U-shaped cross-section with a base connected to said shaft and two free legs (6,7) forming a channel (4) for receiving a rod;
a rod (9) having an at least partially structured surface (10, 11);
a locking member (12) cooperating with the receiving part for locking said rod in the channel (4);
wherein at least on the locking member (12) or in the receiving part an engagement portion (13; 14, 15) for engaging said structured surface (10, 11) of the rod is provided;
characterized in that
the locking member, the rod and the receiving part cooperate in such a manner that as soon as the engagement portion comes into engagement with the structured surface the rod is held in the receiving part in a form-locking manner preventing movement of the rod.

## Description

The invention relates to a bone anchoring assembly for use in spinal surgery or trauma surgery. In particular, the invention relates to a bone anchoring assembly comprising at least two bone anchoring elements with a receiving part to receive a rod and such a rod for connecting the bone anchoring elements wherein the rod is fixed in the receiving part by means of a form-locking connection.

US 5,207,678 discloses a pedicle screw comprising a screw and a receiver member to connect the screw with a rod. The rod is a threaded rod which is locked in the receiver member by means of two nuts engaging the outside of the receiver member and being adjustable on the threaded rod. For re-adjusting the position of the rod relative to the receiver member the nuts are rotated on the threaded rod. This procedure is time consuming since always two nuts have to be adjusted.

US 5,443,467 discloses a bone anchoring assembly comprising a bone anchoring element with a receiving part for receiving a rod and an inner screw and an outer lock nut for securing and fixing the rod in the receiving part. The rod has a smooth surface. The fixation of the rod is achieved by means of frictional locking. Even in a case in which a temporary locking of the rod is desired for allowing a re-adjustment in a later step, a considerable torque has to be applied to achieve a sufficient clamping force.

US 5,716,356 discloses an anchoring member which is to be connected with a threaded rod. The anchoring member has a shaft to be anchored in the bone and a receiving part with a U-shaped cross-section to receive the rod. By means of the recess two open legs are formed which are provided with an external and an internal thread. A nut member is provided which is to be screwed on the external thread and a locking member is to be screwed between the legs. Either the nut member or the locking member or both have a projection which engages with the threaded rod. The anchoring member further comprises a pre-loaded spring member on the bottom of the U-shaped recess acting onto the rod in order to, on the one hand, obtain a rigid connection between the anchoring member and the rod and, on the other hand, to allow an easy manipulation for precisely adjusting the position between the anchoring member and the rod before finally locking the position. Due to the presence of the spring member the rod is still displaceable in its longitudinal direction if the locking member is not yet fully tightened. Also due to the presence of the spring member, the connection between the rod and the receiving part always has a frictional-locking contribution requiring a large torque to be applied for sufficient clamping.

US 5,961,517 discloses an adjustment tool for adjusting the position of the rod in such an anchoring member.

It is the object of the invention to provide a bone anchoring assembly which allows a quick and precise adjustment and re-adjustment of the position of the rod relative to the anchoring member without having to use large torque forces.

The object is solved by a bone anchoring assembly according to claim 1. Further developments are given in the dependent claims.

The bone anchoring assembly has the advantage that it provides a large clamping force on the rod by the application of only a small torque via the locking element when compared to the fixation of a rod via frictional locking. This facilitates a temporary fixation of the rod via the application of small torques on the locking element during the process of adjusting and re-adjusting the position of the rod. Due to the form- locking connection the temporary fixation has sufficient strength to withstand loosening when manipulations are carried out on the rod. Further, the temporary fixation can be loosened quickly by only slightly turning back the inner screw. Therefore, the bone anchoring device is suitable in the application of correcting spinal deformities such as scoliosis which during surgery require re-adjusting the position of the rod several times until it is finally locked.

If the bone anchoring assembly comprises a ratchet-type rod a further advantage is provided in that with the ratchet-type rod a lower torque for fixing is required to achieve the same holding force as compared to a threaded rod or a smooth rod.

Since a pre-fixation of the rod is achieved with the application of a lower torque as compared to the prior art devices a splaying of the legs of the receiving part is reduced.

Further features and advantages of the invention will become apparent and will be best understood by reference to the following detailed description taken into conjunction with the accompanying drawings.

Fig. 1 is a perspective view of a bone anchoring device according to a first embodiment in the assembled state.

Fig. 2 is a side view of the rod and the inner screw of the bone anchoring device of Fig. 1.

Fig. 3 is a sectional view of the bone anchoring device of Fig. 1 the section taken along the rod axis.

Fig. 4 is enlarged view of detail of Fig. 3.

Fig. 5 is a sectional view of the inner screw of Fig. 2.

Fig. 6 is a perspective view of the receiving portion of the bone anchoring device of Fig. 1 to 4 seen from above.

Fig. 7 is a top view of the receiving portion shown in Fig. 6.

Fig. 8 shows a perspective view of a further modification of the receiving portion shown in Fig. 6.

Fig. 9 shows a top view of the receiving portion shown in Fig. 8.

Fig. 10a shows a perspective view of a modification of the receiving portion.

Fig. 10b shows a top view of the receiving portion shown in Fig. 10a.

Fig. 11 shows a side view of the rod and the inner screw for a modification of the bone anchoring device according to the first embodiment.

Fig. 12 shows a detail of a sectional view with the modified rod similar to the section shown in Fig. 4.

Fig. 13 shows an exploded perspective view of a bone anchoring device according to a second embodiment.

Fig. 14 shows a sectional view of the bone anchoring device of the second embodiment in a section along the longitudinal axis of the rod.

Fig. 15 shows a detail of Fig. 14.

Fig. 16 shows perspective view of the pressure element of the bone anchoring device Fig. 13 to 15.

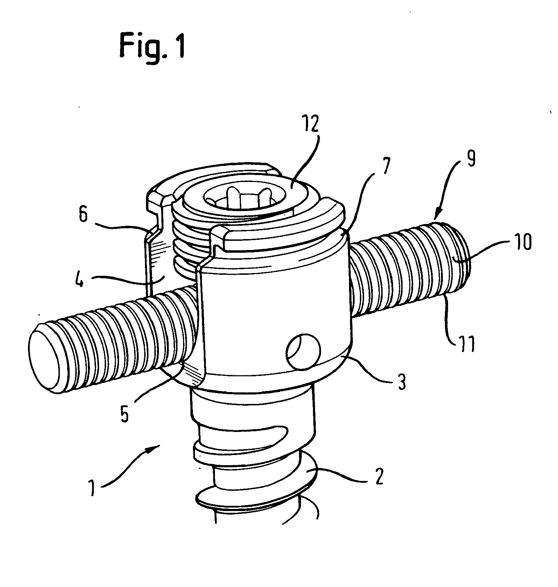

The first embodiment of the anchoring assembly shown in Figs. 1 to 7 comprises a bone anchoring element 1 having a threaded shaft 2 and a receiving part 3. The receiving part 3 is substantially cylindrical and has a U-shaped recess 4 with a bottom 5 at the side of the threaded shaft. By means of the U-shaped recess 4 two free legs 6, 7 are formed. A bore having an internal thread 8 is provided within the channel formed by the U-shaped recess 4 and formed coaxially with the center axis of the threaded shaft 2. The internal thread 8 can have any known thread shape. A flat thread or a negative angle thread however is advantageous in that it prevents splaying of the legs 6, 7.

The anchoring assembly further comprises a longitudinal rod 9 with a structured surface. In the embodiment shown the rod has a circular cross section. The structure is a ratchet-type structure comprising a plurality of circumferential grooves 10 separated by circumferential crests 11.

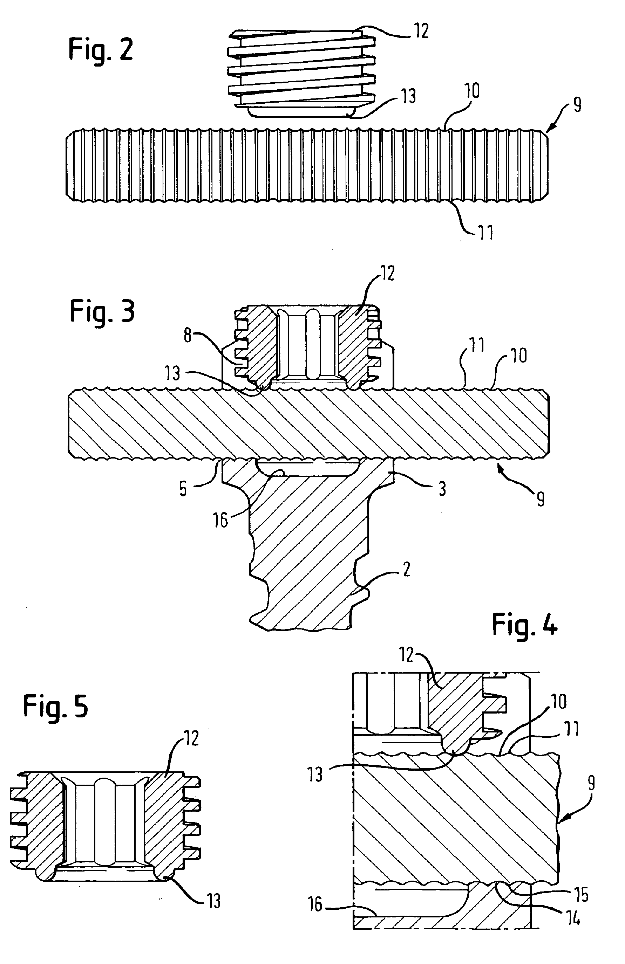

In order to allow the anchoring of the rod 9 in the receiving part a locking member 12 is provided. The locking member in this embodiment is an inner screw or set screw which is to be screwed in the internal thread 8 between the legs 6, 7. The inner screw 12 comprises a ring shaped projection 13 on its side facing the rod as shown in Figs. 2 to 5. The dimensions of the ring shaped projection 13 are selected as a function of the dimensions of the grooves 10 of the rod so that the ring-shaped projection 13 engages the grooves 10 in a position screwed onto the rod, as shown in Fig. 3 and 4. Preferably, the shape of the grooves 10 and the corresponding projection 13 is round.

As shown in Figs. 3, 4 and 6 to 7 the receiving part 3 has on the bottom 5 of its U-shaped recess 4 a structure which can engage the structure of the surface of the rod. In the embodiment shown, the structure comprises a plurality of projections 14 separated by incisions 15 which extend circumferentially in a direction transverse to the longitudinal axis L of the channel which is defined by the recess 4. The projections 14 have dimensions which are selected so as to allow engagement with the grooves 10 of the rod. The number of projections depends on the dimensions of the grooves and the available space on the bottom of the recess.

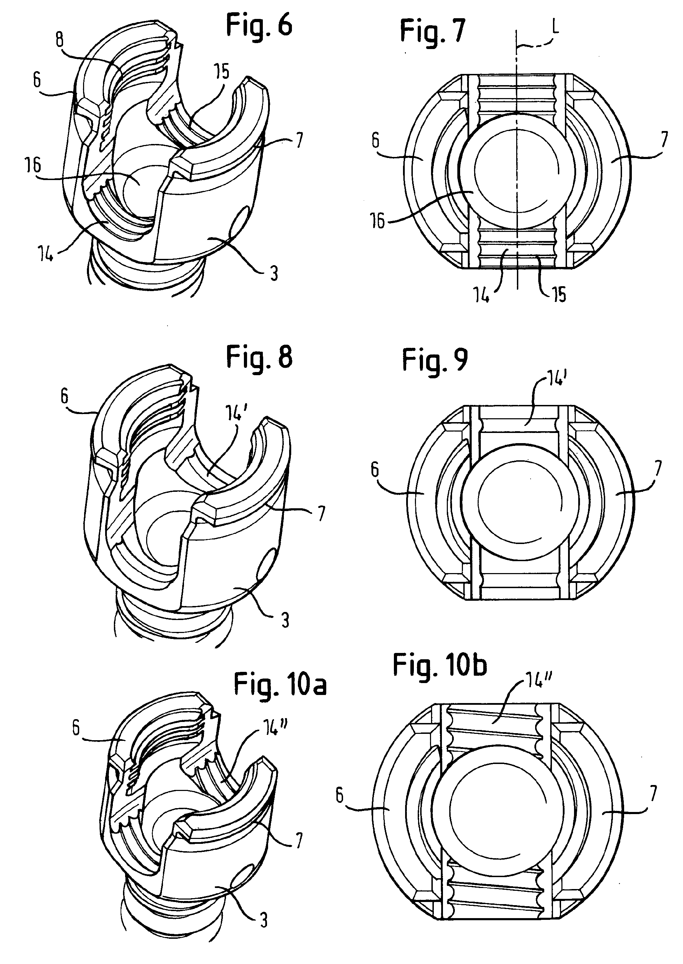

As shown in particular in Figs. 6 and 7, the receiving part further can have a central recess 16 on the bottom of the channel formed by the U-shaped recess 4. By means of the recess 16 the projections 14 are divided into two groups located on the right side and the left side of the recess 16, respectively. This arrangement is advantageous compared to the case in which the projections are provided throughout the bottom 5 of the channel in such a case where a bent rod used.

The incisions 15 between the projections 14 of the structure shown in Figs. 6 and 7 are fine or sharp. In a modification the incisions 15 have a width so as to form valleys between the projections 14. The valleys can be rounded or shaped otherwise. Similarly the structure of the ratchet-type rod can have sharp crests 11 or rounded or otherwise shaped crests 11. The structure used for the ratchet-type rod and the corresponding ring-shaped projection 13 of the inner screw and for the bottom of the receiving part is selected according the material used and/or according to the desired forces acting when the structures come into engagement.

Similarly, the number of projections on the bottom 5 of the receiving part 3 is selected according to the specific requirements. As shown in Figs. 8 and 9 it is possible to provide only one single projection 14' on each side of the recess 16. Also, the inner screw 12 can have more than one single projection to engage with the rod.

The material from which the parts of the bone anchoring assembly are made is a biocompatible material such as, for example, titanium, stainless steel or another material which can be used for bone screws. The material can be different for different elements. In particular, the material is rigid enough to permit a solid form-locking connection between the rod and the bone anchoring element.

In operation, first, at least two bone anchoring elements are screwed into the bone, in particular into adjacent vertebrae. Then, the rod 9 is placed into the U-shaped recesses 4 of the receiving parts of bone anchoring elements such that its surface structure 10, 11 engages with the structure 14, 15 on the bottom of the receiving part. Thereafter, the inner screw 12 is screwed-in. As soon as the ring-shaped projection 13 engages the structure of the surface of the rod, the rod is held in the receiving part in a form-locking manner, i.e. a positive connection, which prevents movement of the rod in any direction. Due to the form-locking or positive locking connection, the application of a low torque is sufficient to obtain a sufficient pre-fixation.

In a following phase fine adjustment is carried out whereby the engagement position of each anchoring element at the rod relative to the longitudinal axis of the rod is adjusted. For fine adjustment, the inner screw is turned back slightly so as to loosen the form-locking engagement of the structure of the rod with the structure of the inner screw. Since the pre-fixation is obtained with low torque, it can be loosened by a quick turning back of the set screw. This facilitates the handling and accelerates the procedure. After the final positions of the anchoring elements relative to the rod are found, the inner screws are tightened so as to firmly fix the rod in the receiving part.

With the anchoring assembly described a pre-fixation of the rod can be obtained with low torque compared to a pre-fixation in the case in which a smooth rod is fixed via frictional locking only. This is also true compared to the case in which a structured rod is fixed using a spring member. Furthermore the anchoring assembly has a simpler construction compared to that having a spring element for clamping the rod. This facilitates the production.

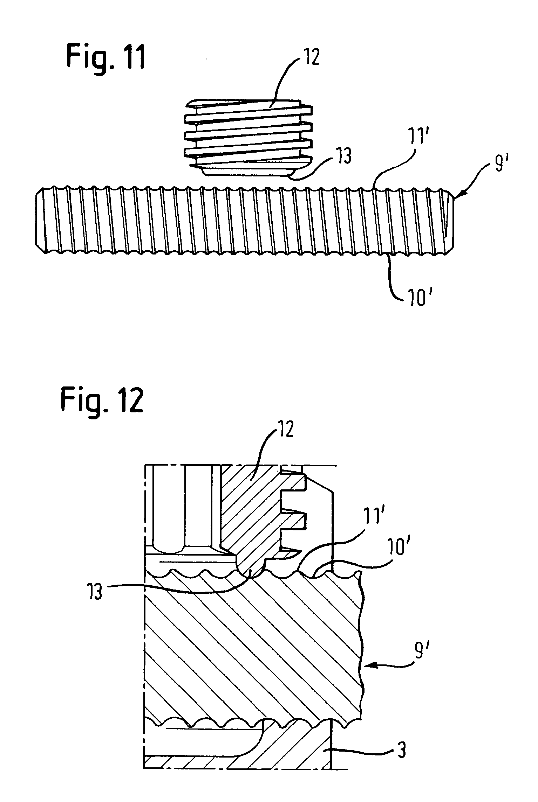

A modification of the first embodiment is shown in Figs. 10a, 10b, 11 and 12. It differs from the first embodiment in that the rod is a threaded rod 9'. Hence, the grooves 10' and the crests 11' have a helical structure with a thread pitch. Correspondingly the structure 14" in the bottom of the receiving part has a thread pitch. The threaded rod 9' is easier to produce compared to the ratchet-type rod.

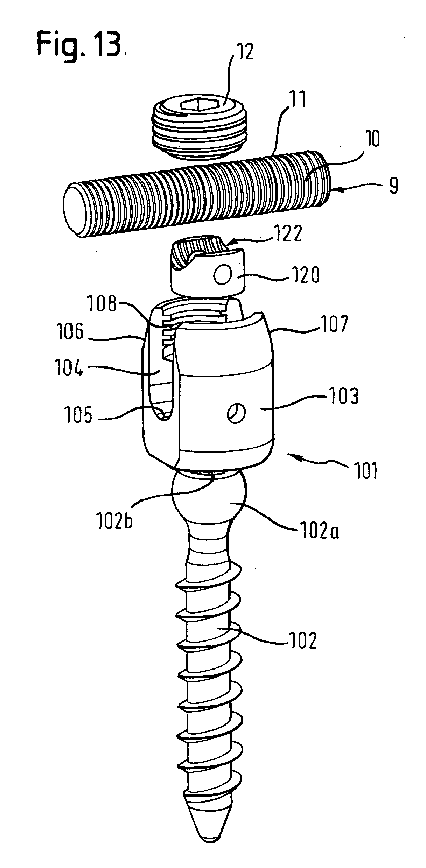

A second embodiment is described with reference to Figs. 13 to 15. The second embodiment differs from the first embodiment in that the bone anchoring element is formed as a so-called polyaxial screw whereby the receiving part receiving the rod is pivotably connected to the associated threaded shaft. The same reference numerals are used for those parts of this embodiment which correspond to the above described embodiments.

The bone anchoring element 101 comprises a threaded shaft 102 and a head 102a having a spherical segment-shaped portion with a recess 102b for engagement with a screwing-in tool. The receiving part 103 is substantially cylindrical and comprises a coaxial bore with a section 103a for receiving the head 102a so that the head is pivotably held in the receiving part. The receiving part 103 further has a U-shaped recess 104 for receiving the rod. By means of the U-shaped recess 104 two legs 106, 107 are formed which comprise an inner thread 108.

A pressure member 120 is provided which is substantially cylindrical and has an outer diameter allowing it to be displaced back and forth in the receiving part. The pressure member has on its side facing the head a, preferably spherical, recess 121 for engagement with at least a part of the head 102a. On the opposite side the pressure member has a cylindrical recess 122 the cylinder axis extending transverse to the screw axis. The radius of the cylindrical recess is adapted to receive the rod 9.

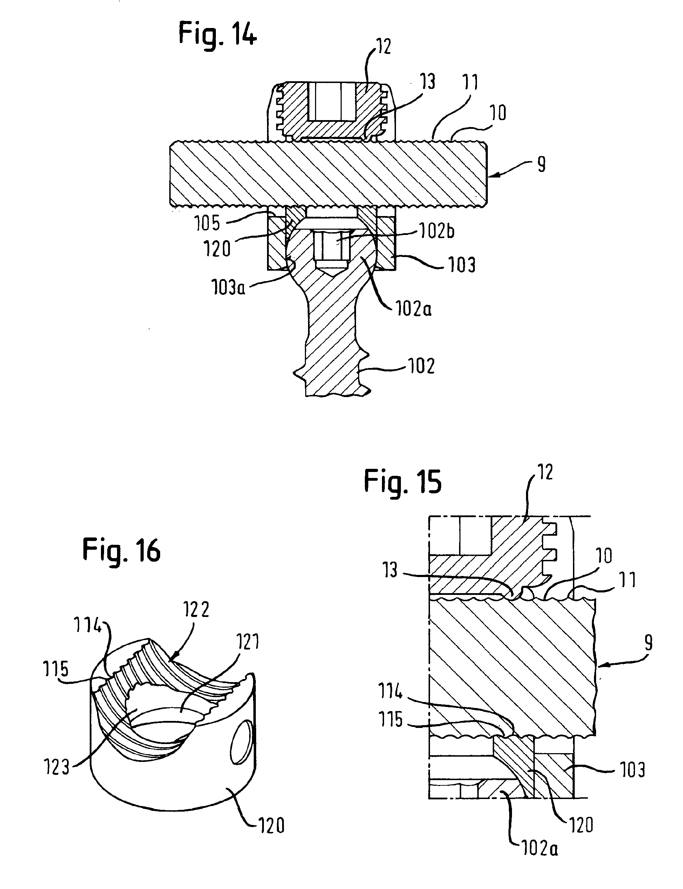

The surface of the cylindrical recess 122 comprises projections 114 separated by incisions 115 extending transverse to the rod axis in a circumferential direction for engagement with the grooves 10 of the rod 9 similar to the structure of the bottom 5 of the receiving part 3 of the first embodiment. The pressure element 120 further has a coaxial bore 123 for permitting access with a screwing-tool. The size of the pressure element is such that in the assembled state the bottom of its cylindrical recess 123 projects above the bottom 105 of the U-shaped recess of the receiving part as shown in Fig. 14 and 15. The screw, the receiving part and the pressure element can be pre-assembled.

The rod 9 and the inner screw 12 are identical to that of the first embodiment.

In operation at least two bone anchoring elements are placed into two bone fragments or two adjacent vertebrae. Thereafter the rod is inserted into the U-shaped recesses of the receiving parts. Since the receiving parts are pivotable with respect to the screws, the angular position of the receiving part relative to the threaded shaft can be adjusted. When the inner screw is screwed-in, it exerts pressure on the rod which exerts pressure on the pressure element to press it against the section 103a of the receiving part. A temporary fixation or pre-fixation can be achieved if the inner screw is tightened with a low torque. The temporary fixation of the rod is caused by the form-locking connection between the inner screw and the rod on the one hand and the rod and the pressure element on the other hand. The fine adjustment is carried out in the same way as with the first embodiment.

Further modifications of the invention are conceivable. The structure of the surface of the rod may vary, for example, it can have wavy helical convolutions. For example sharp crests forming an obtuse angle are also possible. If such sharp crests are provided a finer graduation can be achieved since the formed teeth are smaller in the longitudinal direction of the rod. However, with a rounded shape the fine adjustment can be performed more reliable. The structure of the bottom of the receiving part and of the projection provided at the locking element can also vary.

It is possible to provide the structure only on one part, either on the locking member or on the receiving part or the pressure element, respectively.

In a further modification the U-shaped recess is not open to the top of the receiving part but to the lateral side.

In a further modification of the second embodiment the head and the rod can be fixed independently. In this case the pressure element has instead of the cylindrical recess a U-shaped recess by means of which legs are formed which extend above the rod. The locking member comprises a first screw pressing on the pressure element only and having a threaded coaxial bore in which a set screw is provided which comes into engagement with the rod.

The invention is not limited to screws as bone anchoring elements but can be realized with bone hooks or any other bone anchoring element.

## Claims

1. Bone anchoring assembly for use in spinal surgery or trauma surgery comprising
an anchoring element (1; 101) having a shaft (2; 102) and a receiving part (3; 103) having a substantially U-shaped cross-section with a base connected to said shaft and two free legs (6,7; 106, 107) forming a channel (4; 104) for receiving a rod;
a rod (9) having an at least partially structured surface (10, 11; 10', 11');
a locking member (12) cooperating with the receiving part for locking said rod in the channel;
wherein at least on the locking member (12) or in the receiving part an engagement portion (13; 14, 15; 114, 115) for engaging said structured surface (10, 11; 10', 11') of the rod is provided;
**characterized in that**
the locking member, the rod and the receiving part cooperate in such a manner that as soon as the engagement portion comes into engagement with the structured surface the rod is held in the receiving part in a form-locking manner preventing movement of the rod.

2. The bone anchoring assembly of claim 1, wherein the engagement portion (13; 14, 15; 114, 115) is provided on the locking member (12) and in the receiving part (3; 103).

3. The bone anchoring assembly of claim 1 or 2, wherein the structured surface comprises grooves (10) extending in a circumferential direction of said rod.

4. The bone anchoring assembly of claim 1 or 2, wherein said structured surface comprises a thread (10') or wavy helical convolutions.

5. The bone anchoring assembly of one of claims 1 to 3,
wherein the engagement portion comprises a projecting edge (13).

6. The bone anchoring assembly of one of claims 3 to 5,
wherein the engagement portion comprises a plurality of projections (14; 114) mating with the structure (10, 10') of the structured surface.

7. The bone anchoring assembly of one of claims 1 to 6,
wherein the receiving part (3; 103) comprises an internal thread (8; 108) provided on said legs and the locking element is an inner screw (12) to be screwed-in between said legs.

8. The bone anchoring assembly of one of claims 1 to 7,
wherein the receiving part (3) and the shaft (2) are rigidly connected and said engagement portion (14) is provided on the bottom (5) of the channel (4).

9. The bone anchoring assembly of one of claims 1 to 7,
wherein the receiving part (103) and the shaft (102) are pivotably connected.

10. The bone anchoring assembly of claim 9, wherein the shaft comprises a head (102a) on one end which is held in the receiving part (103) and a pressure element (120) is provided for exerting a pressure on the head to lock the head in the receiving part.

11. The bone anchoring assembly of claim 10, wherein the pressure element (120) comprises the engagement portion (114, 115).
